# FASCICULE DE BREVET EUROPEEN

(11) **EP 1 284 766 B1**
(45) Date de publication et mention de la délivrance du brevet: **23.03.2005**
(21) Numéro de dépôt: 01938334.8
(22) Date de dépôt: 23.05.2001
(51) Int. Cl.: A61M 5/34

(54) **DISPOSITIF DE CONNEXION ETANCHE A USAGE MEDICAL, DU TYPE "LUER-LOCK", ET SERINGUE INCORPORANT UN TEL DISPOSITIF**
ABDICHTENDE LUER-LOCK VERBINDUNGSVORRICHTUNG ZUM MEDIZINISCHEN GEBRAUCH UND SPRITZE AUSGESTATTET MIT EINER SOLCHEN VORRICHTUNG
LUER-LOCK TYPE SEALING CONNECTION DEVICE FOR MEDICAL USE, AND SYRINGE COMPRISING SAME

(30) Priorité: 26.05.2000 FR 0006793
(43) Date de publication de la demande: 26.02.2003
(73) Titulaire: Becton Dickinson France, 38800 Le Pont-de-Claix (FR)
(72) Inventeur: THIBAULT, Jean-Claude, F-38260 La Côte St André (FR); JANSEN, Hubert, D-35041 Marburg-Michelbach (DE); GRIMARD, Jean-Pierre, F-38450 Vif (FR)
(74) Mandataire: Guerre, Dominique
(86) Numéro de dépôt international: PCT/FR2001/001615
(87) Numéro de publication internationale: WO 2001/091839

(56) Documents cités:
- EP-A- 0 716 860
- EP-A- 1 080 742
- DE-A- 19 956 243
- US-A- 4 589 871
- US-A- 5 851 201

## Description

La présente invention se rapporte aux dispositifs de connexion à usage médical, et plus particulièrement aux dispositifs du type "luer-lock".

Le dispositif concerné par la présente invention comprend d'une manière générale :
a) un embout dans lequel est ménagé un passage axial ; cet embout comporte une portée de jonction, par exemple une portée, distale externe d'emmanchement par friction, par exemple de forme frusto-conique, et une zone annulaire proximale de retenue, consistant en une gorge, à fond plat par exemple ;
b) une collerette rapportée, ménageant un logement ouvert axial et distal, dont la paroi comporte une partie distale avec un taraudage intérieur, et une partie proximale dans laquelle une ouverture proximale centrale est ménagée ; le diamètre de cette ouverture est ajusté avec celui de la zone annulaire proximale de l'embout ; la collerette est fixée ou montée en translation et/ou rotation sur l'embout, par engagement entre la zone annulaire proximale de retenue de l'embout, d'une part, et l'ouverture proximale centrale de retenue, d'autre part ;
c) un organe de connexion comportant une autre portée de jonction complémentaire, par exemple une portée interne d'emmanchement par friction, adaptée à la portée de jonction de l'embout ; cet organe comporte au moins une ailette externe proximale et transversale, formant un filet adapté au taraudage intérieur de la collerette, avec un conduit axial en communication fluide avec l'intérieur de l'organe de connexion.

Un tel dispositif est plus particulièrement associé à une seringue, qui comporte, par ailleurs, de manière traditionnelle, un corps creux formant réservoir pour un liquide à éjecter de ou prélevé dans ladite seringue, un joint d'étanchéité disposé à l'intérieur du corps pour délimiter ledit réservoir, et une tige d'actionnement du joint formant piston, disposée dans le reste du réservoir avec une partie proximale de préhension.

Lorsqu'un dispositif de connexion tel que précédemment défini est associé par exemple à une seringue :
- d'une part, l'extrémité distale du corps formant réservoir est conformée selon l'embout défini au paragraphe ci-dessus a) de la définition générale précédente, la collerette étant montée ou fixée sur cet embout, comme défini au paragraphe b) ci-dessus de la définition générale précédente,
- d'autre part, un ensemble d'aiguille formant l'organe de connexion tel que défini au paragraphe c) ci-dessus de la définition générale précédente, l'aiguille formant alors le conduit axial en communication fluide avec l'intérieur dudit organe.

En général, une seringue telle que précédemment définie est fournie à l'utilisateur, par exemple du personnel médical, en deux parties, à savoir :
- une première partie consistant en la seringue proprement dite, en verre ou en matériau plastique, jetable ou réutilisable ; pré-remplie ou non ;
- et l'ensemble d'aiguille, tel que défini précédemment, contenu au moins partiellement dans une coiffe de protection, afin d'éviter tout contact accidentel de la pointe d'aiguille avec l'utilisateur ; des moyens disposés entre l'extérieur du capuchon et l'intérieur de la coiffe permettent de caler l'ensemble d'aiguille en rotation par rapport à la coiffe, ces moyens pouvant consister en des nervures longitudinales coopérant avec des rainures longitudinales ; la coiffe peut comporter par ailleurs, à sa partie proximale, une cuvette de réception de la collerette montée et fixée sur l'embout de la seringue.

La manipulation de ces deux composants s'effectue comme suit.

L'utilisateur rapproche la cuvette de la coiffe de la collerette de la seringue, et par conséquent, le capuchon du logement axial de la collerette, et ce, de manière alignée selon un axe commun à la seringue avec sa collerette, l'ensemble d'aiguille, et la coiffe de protection.

Par vissage de l'allette externe du capuchon de l'ensemble de seringue dans le logement axial de la collerette, on provoque un emmanchement par friction entre la portée distale interne du capuchon et la portée distale externe de l'embout ; un tel emmanchement ne peut être désolidarisé par traction de l'ensemble d'aiguille par rapport à la seringue proprement dite, compte tenu de l'assemblage par vissage entre la collerette et le capuchon.

Une fois le vissage terminé, et donc l'emmanchement par friction effectué, on peut, par simple traction, retirer la coiffe de protection.

Le document DE 19 956 243 décrit un dispositif de connexion du type "Luer-Lock" à usage médical comprenant un embout, une collerette rapportée sur cet embout et un organe de connexion.

Le document EP 01 080 742 décrit un dispositif de type "Luer-Lock " comprenant un cylindre extérieur et un cylindre intérieur moulés en une seule pièce, tout ou partie de la surface de l'espace cylindrique formé entre les deux cylindres pouvant avoir subi un traitement de surface afin de la rendre rugueuse.

La présente invention a pour objet d'améliorer la connexion "luer-lock" précédemment définie, et plus précisément d'éviter toute déconnexion intempestive, en toute circonstance, en particulier lorsque tout ou partie du dispositif de connexion entre en contact avec un matériau liquide ou huileux.

Conformément à la présente invention, on a découvert que, lorsque la zone annulaire proximale de retenue de l'embout, et/ou l'ouverture proximale centrale de retenue de la collerette comportent une bande ou cordon circulaire revêtu par un agent de rugosité, alors, il devient difficile, voire impossible de mettre en rotation la collerette par rapport à l'embout, au moment du vissage entre le capuchon et ladite collerette, d'une part, de désengager la collerette par rapport à l'embout, lorsque la coiffe de protection est extraite du capuchon, d'autre part.

La présente invention comporte encore les modes d'exécution suivants.

Préférentiellement, la zone annulaire proximale de retenue de l'embout est une gorge à fond plat, et l'ouverture centrale de retenue de la collerette forme un chant circulaire, adapté pour s'encliqueter dans cette gorge ; dans ce cas, par exemple, l'agent de rugosité revêt le fond plat de la gorge et/ou le chant de l'ouverture centrale.

L'agent de rugosité comprend des particules d'un matériau inerte et abrasif, par exemple en céramique, de préférence incrustées et liées à la surface de la bande circulaire précitée.

Les particules du matériau inerte et abrasif sont préférentiellement liées par un liant revêtissant la bande circulaire.

L'agent de rugosité, sous forme de particules, peut être déposé ou apporté sous forme bande ou cordon circulaire, ou dans la zone annulaire proximale de retenue de l'embout, ou dans l'ouverture proximale centrale de retenue de la collerette, par tout moyen approprié, tel que projection sous forme de poudre, par exemple poudrage électrostatique, brossage, etc.

Mais, on préférera tout procédé consistant à former une solution ou suspension du liant dans un milieu liquide approprié, à revêtir la bande circulaire précitée avec le liant et les particules de l'agent de rugosité, en suspension ou solution, puis à éliminer le milieu liquide. A cet égard, et à toutes fins utiles, on se référera aux procédés décrits dans le document US-C-4 589 871.

L'agent de rugosité peut être appliqué sur la surface de la bande de contact entre la collerette et l'embout, selon tout motif approprié, par exemple de manière continue, par bandes ou points.

L'agent de rugosité peut être un moyen physique permettant d'obtenir la rugosité recherchée sur la bande circulaire de contact entre la collerette et l'embout. Un tel moyen physique peut être une abrasion avec un flux d'air sous pression entraînant des particules abrasives, ou un faisceau laser.

La présente invention est maintenant décrite par référence au dessin annexé, dans lequel :

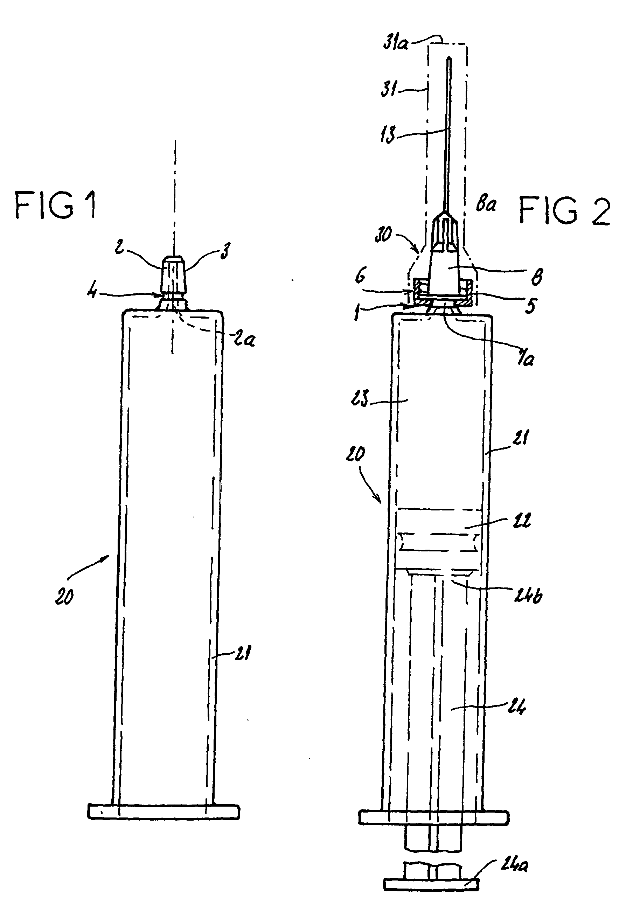

- **la figure 1** représente une seringue de type "luer-lock", non connectée à un ensemble d'aiguille ;
- **la figure 2** représente la même seringue connectée à un ensemble d'aiguille, protégée par une coiffe de protection ;
- **la figure 3** représente le dispositif de connexion proprement dit, en vue axiale, avant assemblage ;
- **la figure 4** représente le même dispositif, toujours en vue axiale, une fois la connexion effectuée entre, d'un côté l'embout et la collerette, et de l'autre côté le capuchon muni du conduit axial ou aiguille.

La présente invention est maintenant décrite par référence à une seringue comportant de manière générale :
- une seringue 20 proprement dite ;
- et un ensemble d'aiguille 30, pourvu d'une coiffe de protection 31.

La seringue 20 comporte de manière traditionnelle :
- un corps 21, obtenu en verre ou en une matière plastique ;
- un joint 22 d'étanchéité formant piston, et délimitant avec le reste du corps 21 un réservoir 23 de volume variable, pour le liquide ou fluide à éjecter ou prélever ;
- une tige 24 disposée coaxialement au corps 21, comportant une extrémité proximale 24a de manipulation, et une extrémité distale 24b fixée sur le joint 22, par exemple par vissage.

L'ensemble d'aiguille 30 comprend :
- un organe de connexion 8, dont la partie distale forme un moyen 8a avec des nervures longitudinales 8b de calage en rotation.
- une aiguille ou conduit axial 13 solidarisée sur le capuchon, en communication fluide avec l'intérieur de ce dernier ;
- une coiffe 31 de protection, fermée à son extrémité distale 31a, déterminant un passage axial 31a pour l'aiguille 13, et pourvue de rainures longitudinales 31 b respectivement pour la réception ou emboîtement des nervures 8b la coiffe comporte par ailleurs une partie proximale évasée 31a.

Le dispositif 1 de connexion proprement dit, associant d'un côté l'ensemble d'aiguille 30, et de l'autre côté la seringue 20 pourvue de sa collerette 5, est maintenant décrit.

Ce dispositif de connexion étanche comprend donc :
a) un embout 2 formant l'extrémité axiale du corps 21 de seringue 20; dans lequel est ménagé un passage interne axial 2a ; cet embout comporte une portée distale externe 3 de jonction étanche, à savoir d'emmanchement par friction, par exemple de forme frusto-conique, et une zone annulaire proximale 4 de retenue, consistant par exemple en une gorge à fond plat 4a ;
b) une collerette 5, par exemple en matière plastique injectée, ménageant un logement axial 5a ouvert ; la paroi 6 de cette collerette comporte une partie distale 6a cylindrique, avec un taraudage intérieur 12, et une partie proximale 6b dans laquelle est ménagée une ouverture proximale centrale 7 ; cette ouverture centrale de retenue forme un chant 7a circulaire, dont le diamètre est ajusté avec celui de la zone annulaire proximale 4 de l'embout 2, ce qui permet un encliquetage de la collerette 5 dans la zone annulaire proximale 4 de retenue, ou gorge à fond plat, de l'embout 2 ; ainsi, la collerette 5 se trouve fixée en translation et/ou en rotation sur l'embout 2, par engagement entre cette zone annulaire 4, ou gorge, et l'ouverture proximale centrale 7, et plus précisément le chant 7a de la collerette 5 ;
c) un organe de connexion 8 comportant une autre portée 9 de jonction complémentaire, interne, par exemple de forme frusto-conique, à savoir d'emmanchement par friction, adaptée à la portée distale 3 externe d'emmanchement de l'embout 2 ; cet organe comporte par ailleurs deux ailettes externes et radiales 10, proximales et transversales, formant ensemble un filet 11 adapté au taraudage intérieur 12 de la collerette 5 ; et comme indiqué précédemment, l'aiguille 13 axiale est en communication fluide avec l'intérieur de l'organe de connexion 8.

Conformément à la présente invention, la zone annulaire proximale 4 de retenue de l'embout 2, par exemple gorge à fond plat 4a, et/ou l'ouverture centrale 2 de retenue de la collerette 5, ou le chant 7a circulaire, comportent une bande circulaire 4a revêtue avec ou traitée par un agent de rugosité ; préférentiellement, cet agent de rugosité revêt le fond plat 4a de la gorge, et/ou le chant 7a de l'ouverture centrale 7.

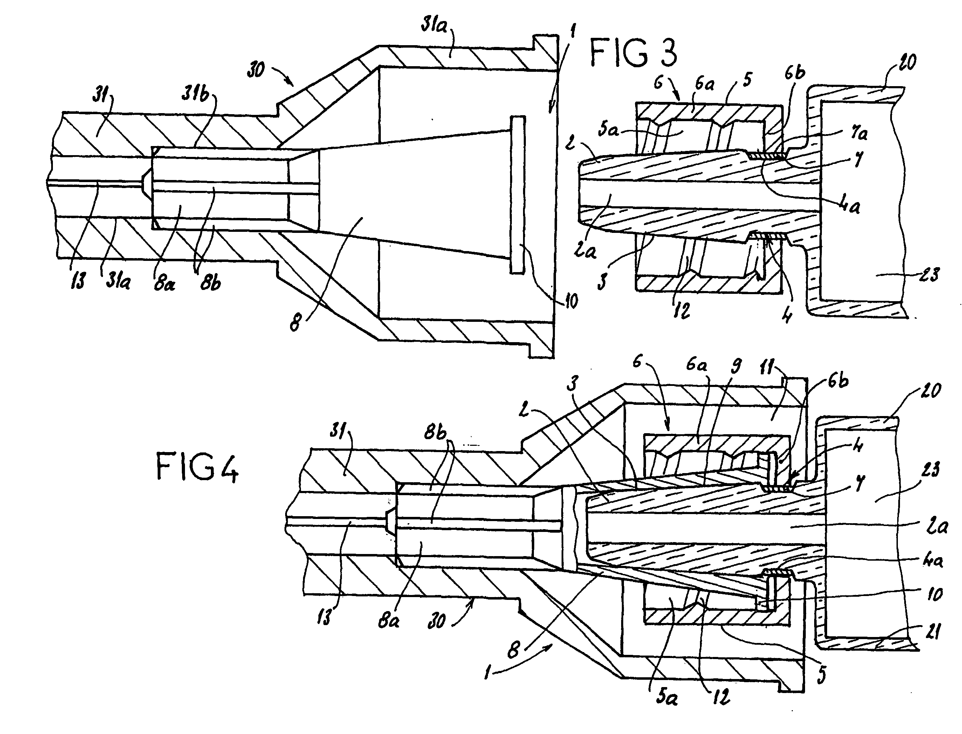

Le fonctionnement du dispositif de connexion se déduit de la comparaison des représentations des figures 3 et 4, et a été explicité dans le préambule de la présente description.

Grâce à l'agent de rugosité, on obtient une connexion complète et assurée de l'ensemble d'aiguille 30 sur l'embout 2 de la seringue 20.

Différentes variantes de la solution selon l'invention peuvent être considérées :
- la zone annulaire proximale 4 de retenue de l'embout peut être différente d'une gorge ; dans le cas d'une gorge, celle-ci peut être à fond concave ou anguleux,
- l'agent de rugosité peut être un dépolissage d'une surface originellement lisse, par abrasion, attaque chimique et aussi un adhésif déposé par tout moyen approprié.

## Revendications

1. Dispositif de connexion à usage médical, du type "luer-lock", comprenant :
a) un embout (2) dans lequel est ménagé un passage interne axial (2a), comportant une portée de jonction (3), et une zone annulaire proximale (4) de retenue ;
b) une collerette (5) ménageant un logement ouvert axial (5a) dont la paroi (6) comporte une partie distale (6a) avec taraudage intérieur (12) et une partie proximale (6b) transversale dans laquelle une ouverture proximale centrale de retenue (7) est ménagée et dont le diamètre est ajusté avec celui de ladite zone annulaire proximale (4) de l'embout (2), ladite collerette (5) étant fixée en translation et/ou rotation sur l'embout (2), par engagement entre la zone annulaire proximale de retenue (4) dudit embout et l'ouverture proximale centrale (7) de retenue ;
c) un organe de connexion (8) comportant une autre portée de jonction complémentaire (9), adaptée à la portée (3) de jonction de l'embout (2), et au moins une ailette externe (10) proximale et transversale, formant un filet (11) adapté au taraudage intérieur (12) de la collerette (5), avec un conduit axial (13) en communication fluide avec l'intérieur dudit organe de connexion (8).
**caractérisé en ce que** la zone annulaire proximale (4) de retenue de l'embout (2) et/ou l'ouverture proximale centrale (7) de retenue de la collerette (5) comportent une bande ou cordon circulaire (4a) revêtu ou traité par un agent de rugosité.

2. Dispositif selon la revendication 1, **caractérisé en ce que** la zone annulaire proximale (4) de retenue de l'embout (2) est une gorge, à fond plat (4a) et l'ouverture centrale (7) de retenue de la collerette (5) forme un chant (7a) circulaire adapté pour s'encliqueter dans ladite gorge.

3. Dispositif selon la revendication 2, **caractérisé en ce que** l'agent de rugosité revêt le fond plat (4a) de la gorge et/ou le chant (7a) de l'ouverture centrale

4. Dispositif selon la revendication 1, **caractérisé en ce que** l'agent de rugosité comprend des particules d'un matériau inerte et abrasif, de préférence incrustées et liées à la surface de la bande circulaire (4a) et/ou (7a).

5. Dispositif selon la revendication 4, **caractérisé en ce que** les particules du matériau inerte et abrasif sont liées par un liant revétissant la bande circulaire (4a) et/ou (7a).

6. Seringue (20) comportant un dispositif de connexion du type "luer-lock" conforme à la revendication 1,

7. Ensemble (30) d'aiguille de seringue, comportant un dispositif de connexion selon la revendication 1.

## Patentansprüche

1. Verbindungsvorrichtung vom Typ "Luer-Lock" zur medizinischen Verwendung, die folgendes aufweist:
a) einen Ansatz (2), in dem eine innere axiale Passage (2a) ausgebildet ist und der einen Anschlußbereich (3) sowie eine ringförmige proximale Halte-rungszone (4) aufweist;
b) einen Flansch (5), der eine offene axiale Aufnahme (5a) darstellt und dessen Wand (6) einen distalen Teil (6a) mit einem Innengewinde (12) sowie ein sich in Querrichtung erstreckendes proximales Teil (6b) aufweist, in dem eine zentrale proximale Halterungsöffnung (7) ausgebildet ist, deren Durch-messer mit demjenigen der ringförmigen proximalen Halterungszone (4) des Ansatzes (2) abgestimmt ist, wobei der Flansch (5) durch den Eingriff zwischen der ringförmigen proximalen Halterungszone (4) des Ansatzes und der zentralen proximalen Halterungsöffnung (7) für eine Translation und/oder eine Rotation an dem Ansatz (2) befestigt ist; und
c)ein Verbindungsorgan (8), welches einen weiteren, komplementären Anschlußbereich (9) aufweist, der an den Anschlußbereich (3) des Ansatzes (2) angepaßt ist, und mindestens einen proximalen und sich in Querrichtung erstreckenden äußeren Steg (10) aufweist, der ein an das Innengewinde (12) des Flansches (5) angepaßtes Gewindeelement (11) bildet, mit einer axialen Leitung (13), die mit dem Inneren des Verbindungsorgans (8) in Fluidverbindung steht, **dadurch gekennzeichnet, daß** die ringförmige proximale Halterungszone (4) des Ansatzes (2) und/oder die zentrale proximale Halterungsöffnung (7) des Flansches (5) einen kreisförmigen Streifen oder ein kreisförmiges Band (4a) aufweist bzw. aufweisen, der bzw. das mit einem Rauhigkeitsmittel beschichtet oder behandelt ist.

2. Vorrichtung nach Anspruch 1, **dadurch gekennzeichnet, daß** die ringförmige proximale Halterungszone (4) des Ansatzes (2) eine Nut mit einem ebenen Grund (4a) ist und daß die zentrale Halterungsöffnung (7) des Flansches (5) einen kreisförmigen Rand (7a) bildet, der zum Einrasten in die Nut ausgelegt ist.

3. Vorrichtung nach Anspruch 2, **dadurch gekennzeichnet, daß** das Rauhigkeitsmittel den ebenen Grund (4a) der Nut und/oder den Rand (7a) der zentralen Öffnung bedeckt.

4. Vorrichtung nach Anspruch 1, **dadurch gekennzeichnet, daß** das Rauhigkeitsmittel Partikel aus einem inerten und abrasiven Material aufweist, welche bevorzugt an der Oberfläche des kreisförmigen Streifens (4a) und/oder (7a) angelagert und damit verbunden sind.

5. Vorrichtung nach Anspruch 4, **dadurch gekennzeichnet, daß** die Partikel aus inertem und abrasivem Material durch ein Bindemittel gebunden sind, welches den kreisförmigen Streifen (4a) und/oder (7a) bedeckt.

6. Spritze (20), welche eine Verbindungsvorrichtung vom Typ "Luer-Lock" gemäß Anspruch 1 aufweist.

7. Spritzennadeleinheit (30), welche eine Verbindungsvorrichtung gemäß Anspruch 1 aufweist.

## Claims

1. Connection device for medical use, of the Luer lock type, comprising:
a) a nozzle (2) in which an axial internal passage (2a) is formed, including a bearing connection surface (3), and a proximal annular retention zone (4);
b) a flange (5) forming an axial open seat (5a) whose wall (6) includes a distal part (6a) with internal screw thread (12) and a transverse proximal part (6b) in which a central proximal retention opening (7) is formed, and whose diameter is matched to that of the said proximal annular zone (4) of the nozzle (2), the said flange (5) being fixed in translation and/or rotation on the nozzle (2), by engagement between the proximal annular retention zone (4) of the said nozzle and the central proximal retention opening (7);
c) a connection member (8) including another complementary bearing connection surface (9), matching the bearing connection surface (3) of the nozzle (2), and at least one proximal and transverse external wing (10), forming a thread (11) matching the internal screw thread (12) of the flange (5), with an axial conduit (13) in fluid communication with the inside of the said connection member (8),
**characterized in that** the proximal annular retention zone (4) of the nozzle (2) and/or the central proximal retention opening (7) of the flange (5) include a circular band or cord (4a) covered or treated with a roughening agent.

2. Device according to Claim 1, **characterized in that** the proximal annular retention zone (4) of the nozzle (2) is a groove, with a flat bottom (4a), and the central retention opening (7) of the flange (5) forms a circular edge (7a) designed to snap into the said groove.

3. Device according to Claim 2, **characterized in that** the roughening agent covers the flat bottom (4a) of the groove and/or the edge (7a) of the central opening.

4. Device according to Claim 1, **characterized in that** the roughening agent comprises particles of an inert and abrasive material, preferably encrusted and bound to the surface of the circular band (4a) and/or (7a).

5. Device according to Claim 4, **characterized in that** the particles of the inert and abrasive material are bound by a binder covering the circular band (4a) and/or (7a).

6. Syringe (20) including a connection device of the Luer lock type according to Claim 1.

7. Syringe needle assembly (30), comprising a connection device according to Claim 1.
